(19) Europäisches Patentamt
European Patent Office
Office européen des brevets

(11) EP 3 752 138 B1

(12) **FASCICULE DE BREVET EUROPEEN**

(45) Date de publication et mention de la délivrance du brevet:
**09.02.2022 Bulletin 2022/06**

(21) Numéro de dépôt: **19710047.2**

(22) Date de dépôt: **13.02.2019**

(51) Classification Internationale des Brevets (IPC):
***A61K 31/167*** (2006.01) ***A61P 25/28*** (2006.01)
***A61P 25/02*** (2006.01) ***A61P 25/00*** (2006.01)
***A61P 25/16*** (2006.01) ***A61P 9/14*** (2006.01)
***A61P 11/00*** (2006.01) ***A61P 3/10*** (2006.01)
***C07C 235/42*** (2006.01) ***C07C 235/58*** (2006.01)

(52) Classification Coopérative des Brevets (CPC):
**A61K 31/167; A61P 3/10; A61P 9/14; A61P 11/00; A61P 25/00; A61P 25/02; A61P 25/16; A61P 25/28; C07C 235/64; C07C 235/66**

(86) Numéro de dépôt international:
**PCT/FR2019/050321**

(87) Numéro de publication internationale:
**WO 2019/158859 (22.08.2019 Gazette 2019/34)**

(54) **COMPOSÉS ET COMPOSITIONS POUR LE TRAITEMENT DE MALADIES NEURODÉGÉNÉRATIVES ET INFLAMMATOIRES**

VERBINDUNGEN UND ZUSAMMENSETZUNGEN ZUR BEHANDLUNG VON NEURODEGENERATIVEN UND ENTZÜNDLICHEN ERKRANKUNGEN

COMPOUNDS AND COMPOSITIONS FOR THE TREATMENT OF NEURODEGENERATIVE AND INFLAMMATORY DISEASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.02.2018 FR 1851206**

(43) Date de publication de la demande:
**23.12.2020 Bulletin 2020/52**

(73) Titulaires:
- **SORBONNE UNIVERSITE**
  **75006 Paris (FR)**
- **Centre National de la Recherche Scientifique**
  **75016 Paris (FR)**
- **Institut National de la Santé et de la Recherche Médicale**
  **75013 Paris (FR)**
- **Assistance Publique Hôpitaux de Paris**
  **75004 Paris (FR)**
- **Institut du Cerveau et de la Moelle Epiniere**
  **75013 Paris (FR)**
- **UNIVERSITE DE MONTPELLIER**
  **34090 Montpellier (FR)**
- **Ecole Nationale Supérieure de Chimie de Montpellier**
  **34090 Montpellier (FR)**

(72) Inventeurs:
- **EL AMRI, Chahrazade**
  **95120 ERMONT (FR)**
- **SOUALMIA, Feryel**
  **94160 SAINT-MANDE (FR)**
- **MASURIER, Nicolas**
  **34070 MONTPELLIER (FR)**
- **AÏT AMIRI, Sabrina**
  **93100 MONTREUIL (FR)**
- **NAIT OUMESMAR, Brahim**
  **75013 PARIS (FR)**
- **DEBOUX, Cyrille**
  **77290 MITRY MORY (FR)**

(74) Mandataire: **Cabinet Becker et Associés**
**25, rue Louis le Grand**
**75002 Paris (FR)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(56) Documents cités:

- **GUYAN LIANG ET AL: "Human kallikrein 6 inhibitors with a-amidobenzylanmine P1 group identified through virtual screening", BIOORGANIC & MEDICINAL CHEMISTRY LETTERS, PERGAMON, AMSTERDAM, NL, vol. 22, no. 7, 6 février 2012 (2012-02-06), pages 2450-2455, XP028471743, ISSN: 0960-894X, DOI: 10.1016/J.BMCL.2012.02.014 [extrait le 2012-02-14]**
- **DATABASE BIOSIS [Online] BIOSCIENCES INFORMATION SERVICE, PHILADELPHIA, PA, US; mars 2003 (2003-03), SCARISBRICK ISOBEL A: "Activity of a newly identified serine protease in central nervous system demyelination.", XP002785206, Database accession no. PREV200300249947 & FASEB JOURNAL, vol. 17, no. 4-5, mars 2003 (2003-03), page Abstract No. 207.1, FASEB MEETING ON EXPERIMENTAL BIOLOGY: TRANSLATING THE GENOME; SAN DIEGO, CA, USA; APRIL 11-15, 2003 ISSN: 0892-6638**
- **DATABASE BIOSIS [Online] BIOSCIENCES INFORMATION SERVICE, PHILADELPHIA, PA, US; juin 2008 (2008-06), SCARISBRICK ISOBEL A ET AL: "Kallikreins are associated with secondary progressive multiple sclerosis and promote neurodegeneration", XP002785207, Database accession no. PREV200800655911 & BIOLOGICAL CHEMISTRY, vol. 389, no. 6, juin 2008 (2008-06), pages 739-745, ISSN: 1431-6730, DOI: 10.1515/BC.2008.085**
- **DATABASE BIOSIS [Online] BIOSCIENCES INFORMATION SERVICE, PHILADELPHIA, PA, US; March 2003 (2003-03), SCARISBRICK ISOBEL A: "Activity of a newly identified serine protease in central nervous system demyelination.", Database accession no. PREV200300249947 & FASEB JOURNAL, vol. 17, no. 4-5, March 2003 (2003-03), page Abstract No. 207.1, FASEB MEETING ON EXPERIMENTAL BIOLOGY: TRANSLATING THE GENOME; SAN DIEGO, CA, USA; APRIL 11-15, 2003 ISSN: 0892-6638**

**Description**

## DOMAINE TECHNIQUE DE L'INVENTION

La présente invention concerne des composés présentant une activité inhibitrice de kallicréines et des compositions comprenant au moins un de ces composés pour une utilisation dans le traitement de maladies ou des désordres présentant un dérèglement de l'activité des kallicréines, en particulier les maladies neurodégénératives et inflammatoires.

## CONTEXTE DE L'INVENTION

Depuis maintenant plus d'une dizaine d'années, l'implication de protéases à sérine dans le fonctionnement du système nerveux central (SNC) est continuellement mise en évidence. Parmi les protéases à sérine relativement nouvelles, les kallicréines tissulaires forment une famille de protéases présente dans au moins six ordres de mammifères. Elles sont impliquées dans des fonctions aussi diverses que la régulation de la plasticité synaptique, la survie des neurones, l'acquisition de la mémoire, et la fonction vasculaire. Parmi les kallicréines tissulaires, la KLK6 (la kallicréine-6 ou hK6) est la plus abondante du SNC adulte. Elle est produite par les neurones et par les oligodendrocytes, et dispose d'une activité protéolytique analogue à celle de la trypsine. La KLK6 possède ainsi une action large et un rôle central dans le cerveau et la moelle épinière. De ce fait, elle est impliquée dans les pathologies touchant le SNC et notamment dans certains processus neurodégénératifs. Malgré les efforts de la communauté scientifique, très peu d'inhibiteurs sont disponibles. Il a ainsi été décrit dans l'article de Liang et al. ("Human kallikrein 6 inhibitors with a para-amidobenzylamine P1 group identified through virtual screening", Bioorganic & Medicinal Chemistry letters, 22 (2012), 2450-2455) des composés avec des structures *para*-amidobenzylamine et 2-hydroxybenzamide présentant une activité inhibitrice de KLK6.

D'autres protéases à sérine que sont la plasmine et la kallicréine 1 (KLK1) ont été également été décrites dans le fonctionnement du SNC.

Ces protéases à serine sont impliquées de manière prépondérante dans les pathologies inflammatoires et neurodégénératives.

Parmi ces pathologies, la sclérose en plaques (SEP) est une maladie démyélinisante autoimmune incurable du SNC. Cette maladie chronique touche plus de deux millions d'individus dans le monde, dont 100 000 en France avec 3000 à 5000 nouveaux cas déclarés chaque année. Chez le jeune adulte, la SEP est le trouble neurologique le plus fréquent ainsi que la première cause de handicap sévère non traumatique. La SEP est associée à une réaction inflammatoire du SNC qui aboutit à une dégradation de la gaine de myéline entourant les axones centraux et donc, à leur démyélinisation. Les conséquences de ce phénomène sont multiples : (i) la transduction de l'influx nerveux est altérée ; (ii) une souffrance neuronale s'installe, allant jusqu'à une neurodégénérescence ; (iii) les débris de myéline aggraveraient le phénomène d'inflammation. Au niveau clinique, ces perturbations se manifestent par des troubles moteurs, sensoriels et cognitifs sévères. Différentes études convergent vers l'hypothèse selon laquelle la KLK6 serait impliquée dans le développement de la SEP. Il est aujourd'hui admis que les patients possèdent un taux anormalement élevé de KLK6 au niveau de leur liquide céphalo-rachidien (LCR). Plus précisément, la KLK6 serait impliquée dans les différentes composantes de la pathologie : la démyélinisation, la neuroinflammation et la dégénérescence axonale. L'excès de KLK6 chez les patients atteints de sclérose en plaques (SEP) entraînerait d'importants phénomènes de neuroinflammation *via* deux voies différentes, celle des récepteurs PARs (Protease Activated Receptors) et celle du clivage excessif de ses substrats. Les médicaments actuellement commercialisés sont des traitements de fond ciblant les aspects inflammatoires de la pathologie. L'identification de nouveaux composés thérapeutiques agissant sur la régénération de la myéline (remyélinisation) dans la SEP est ainsi un véritable enjeu de santé publique. Un effort particulier est mis sur la recherche de composés capables de traiter simultanément l'inflammation et la démyélinisation pour une prise en charge plus complète des patients.

Le but de la présente invention est donc de proposer des modulateurs organiques de ces cibles, et en particulier de la KLK6. La présente invention propose des composés inhibiteurs de protéases à sérine d'intérêt dans les pathologies neurodégénératives et l'inflammation, basés sur une structure *para*-amidobenzylamine. Plusieurs protéases parmi celles les plus importantes du SNC sont ciblées : la plasmine, la kallicréine tissulaire (KLK1), et en particulier la kallicréine 6 (KLK6). La capacité inhibitrice ainsi que le spectre d'action de composés ont été évalués et des inhibiteurs réversibles efficaces de la KLK6 ont ainsi été identifiés. En outre, certains des composés identifiés ne présentent pas de toxicité vis-à-vis de neurones primaires. Ces composés présentent donc un intérêt thérapeutique dans l'axe neuroprotecteur et anti-inflammatoire.

## RESUME DE L'INVENTION

La présente invention propose un composé pour une utilisation dans le traitement d'un désordre ou d'une

maladie liée à un dérèglement de l'activité d'au moins une kallicréine, ledit composé étant de formule (I) suivante :

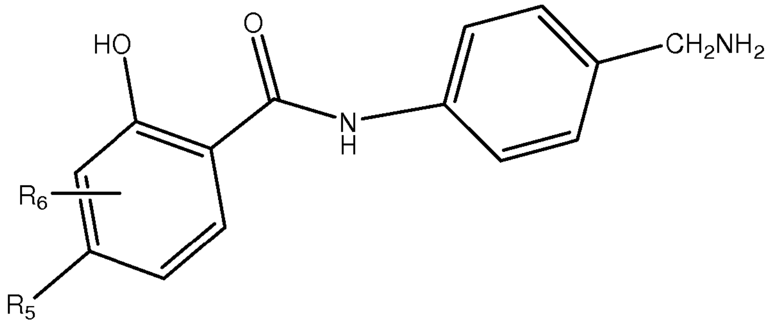

(I)

dans laquelle :

R5 représente un radical (C1-C6)alkyl et R6 représente un atome d'hydrogène ; alternativement, R5 et R6 forment ensemble un cycle avec les deux carbones du cycle phényle auxquels ils sont rattachés pour former un groupe naphtyle, éventuellement substitué par au moins un atome d'halogène, un radical OH, (C1-C6)alkyle, (C1-C6)alkoxy, $-C(O)_2R$, ou -N(R)C(O)R', où R et R' étant indépendamment un atome d'hydrogène ou un radical (C1-C6)alkyl, la fonction amine pouvant présenter un groupement aminoprotecteur, ou un de ses sels pharmaceutiquement acceptable.

La présente invention propose également des compositions, en particulier des compositions pharmaceutiques, comprenant au moins un composé de formule (I), dans un support acceptable sur le plan pharmaceutique, éventuellement en association avec au moins un autre actif thérapeutique.

Elle concerne aussi l'utilisation d'au moins un composé de formule (I) pour la préparation d'une composition pharmaceutique destinée à traiter un désordre ou une maladie liée à un dérèglement de l'activité d'au moins une kallicréine, tel que la sclérose en plaques.

Elle concerne aussi des composés ou des compositions selon l'invention pour une utilisation en tant que médicaments, en particulier pour traiter des maladies neurodégénératives, comme la sclérose en plaques.

Est également divulguée une méthode de traitement d'un désordre ou d'une maladie liée à un dérèglement de l'activité d'au moins une kallicréine d'un sujet, comprenant l'administration audit sujet d'au moins un composé de formule (I) ou une composition comprenant ledit composé.

## DESCRIPTION DETAILLEE DE L'INVENTION

La présente invention concerne des composés de formule (I) telle que définie ci-dessus, pour une utilisation dans le traitement d'un désordre ou d'une maladie liée à un dérèglement de l'activité d'au moins une kallicréine.

Selon la présente invention, le terme "alkyle" désigne un radical hydrocarboné saturé, linéaire, ramifié ou cyclique, halogéné ou non, ayant plus particulièrement de 1 à 6, de préférence 1 à 4, atomes de carbone. Ainsi, parmi les radicaux alkyle, on peut notamment citer le radical méthyle, éthyle, propyle, isopropyle, cyclopropyle, butyle, isobutyle, *tert*-butyle, pentyle, néopentyle, n-hexyle ou cyclohexyle. Les groupes comportant un ou deux atomes de carbone ou comportant de 1 à 4 atomes de carbone sont particulièrement préférés. Les groupes méthyle et éthyle sont tout particulièrement préférés. Les radicaux alkyle peuvent être halogénés, et en particulier perhalogénés, tels que CF3.

Le terme alkyloxy ou alkoxy fait référence à une chaîne alkyle liée au reste de la molécule par l'intermédiaire d'un atome d'oxygène. La chaîne alkyle répond à la définition précédemment énoncée.

Selon un mode particulier, les composés sont de formule (I) dans laquelle R5 représente un radical alkyle de 1 à 6, de préférence 1 à 4, atomes de carbone. De préférence, R5 est choisi parmi un radical méthyle, éthyle, n-propyle, isopropyle, n-butyle, et tert-butyle, en particulier méthyle.

Selon un autre mode particulier, R5 et R6 forment ensemble un cycle avec les deux carbones adjacents du cycle phényle auxquels ils sont rattachés pour former un groupe naphtyle, éventuellement substitué par au moins un atome d'halogène, un radical OH, (C1-C6)alkyle, (C1-C6)alkoxy, $-C(O)_2R$, ou -N(R)C(O)R', où R, et R' étant indépendamment H ou $(C_1-C_6)$alkyl, de préférence au moins un radical (C1-C6)alkyle, (C1-C6)alkoxy ou un atome d'halogène.

Les termes alkyle et alkoxy sont tels que définis ci-dessus.

Par atome d'halogène, on entend un atome de chlore, fluor, brome ou iode.

Selon un autre mode particulier, le composé de formule (I) présente un groupe aminoprotecteur sur la fonction amine.

Par groupement aminoprotecteur, on entend un groupement du type de ceux employés en synthèse peptidique, tels que par exemple les groupes acyle, comme formyle, acétyle, propionyle, phénylacétyle, phénoxyacétyle, etc.; un groupe alkoxycarbonyle, tel que tert-butoxycarbonyl (BOC), etc.; un groupe alkoxyalkylcarbonyle, tel que méthoxypropionyle, etc ; un groupe alkoxycarbonyle substitué, tel que trichloroéthoxycarbonyle, etc.; un groupe alkylcarbonyle substitué, tel que monochlorométhylcarbonyle, monochloroéthylcarbonyle, dichlorométhylcarbonyle, trichlorométhylcarbonyle, trichloroéthylcarbonyle, trichloropropylcarbonyle, trifluorométhylcarbonyle, etc ; un groupe arylalkoxycarbonyle, tel que benzyloxycarbonyle, etc ; un groupe arylalkoxycarbonyle substitué, tel que 4-nitrobenzyloxycarbonyle, etc; un groupe benzyle, éventuellement substitué par au moins un radical (C1-C6)alkyle, (C1-C6)alkoxy ou un atome d'halogène.; un groupe diphénylméthyle éventuellement substitué ; un groupe trityle éventuellement substitué, tel que 4-méthoxyphényldiphénylméthyle ou di(4-méthoxyphényl)phénylméthyle.

Les composés de formule (I) comprennent également les isomères optiques et géométriques, les racémates, les tautomères, les sels, les hydrates et les mélanges des composés selon l'invention.

Les composés de formule (I) incluent les sels pharmaceutiquement acceptables, en particulier les sels d'acides inorganiques et organiques. Des exemples représentatifs d'acides inorganiques incluent l'acide chlorhydrique, bromhydrique, iodique, phosphorique, etc. Des exemples représentatifs d'acides organiques incluent l'acide formique, acétique, trichloroacétique, trifluoroacétique, propionique, benzoïque, cinnamique, citrique, fumarique, maléique, méthanesulfonique, etc. D'autres sels d'addition d'acide organique ou inorganique incluent les sels pharmaceutiquement acceptables décrits dans J. Pharm. Sci. 1977, 66, 2, et dans le « Handbook of Pharmaceutical Salts: Properties, Selection, and Use » édité par P. Heinrich Stahl and Camille G. Wermuth 2002. Selon un mode particulier, les sels sont choisis parmi le maléate, le chlorhydrate, le bromhydrate, et le méthanesulfonate.

Selon un mode particulier, le composé utilisé selon la présente invention est choisi parmi : *N*-(4-(aminomethyl)phenyl)-1-hydroxy-2-naphtamide,

*N*-(4-(aminomethyl)phenyl)-3-hydroxy-2-naphtamide,
*N*-(4-(aminométhyl)phényl)-4-méthyl-2-hydroxybenzamide,
*N*-(4-(aminométhyl)phényl)-6-méthoxy-1-hydroxy-2-naphtamide,
*N*-(4-(aminométhyl)phényl)-4-isopropyl-2-hydroxybenzamide,

et un de ses sels, en particulier le chlorhydrate, ou un de ses composés amino-protégés.

Selon un mode plus spécifique, le composé utilisé est choisi parmi le N-(4-(aminométhyl)phényl)-1-hydroxy-2-naphtamide, *N*-(4-(aminométhyl)phényl)-4-méthyl-2-hydroxybenzamide, un de leurs sels, en particulier le chlorhydrate, ou un de leurs composés amino-protégés.

Un autre objet de l'invention est le composé *N*-(4-(aminométhyl)phényl)-4-isopropyl-2-hydroxybenzamide, un de ses sels, en particulier le chlorhydrate, ou un de ses composés amino-protégés.

Un autre objet de la présente invention concerne une composition pharmaceutique comprenant dans un support acceptable sur le plan pharmaceutique au moins un composé de formule (I) tel que décrit ci-dessus, éventuellement en association avec un autre actif thérapeutique.

Selon un mode particulier, une composition pharmaceutique comprenant dans un support acceptable sur le plan pharmaceutique au moins un composé de formule (I) choisi parmi :

*N*-(4-(aminomethyl)phenyl)-1-hydroxy-2-naphtamide,
*N*-(4-(aminomethyl)phenyl)-3-hydroxy-2-naphtamide,
*N*-(4-(aminométhyl)phényl)-4-méthyl-2-hydroxybenzamide,
*N*-(4-(aminométhyl)phényl)-6-méthoxy-1-hydroxy-2-naphtamide,
*N*-(4-(aminométhyl)phényl)-4-isopropyl-2-hydroxybenzamide,

un de ses sels, en particulier le chlorhydrate, ou un de ses composés amino-protégés.

En particulier, la composition pharmaceutique comprend le composé est choisi parmi le *N*-(4-(aminométhyl)phényl)-1-hydroxy-2-naphtamide et *N*-(4-(aminométhyl)phényl)-4-méthyl-2-hydroxybenzamide, plus spécifiquement le *N*-(4-(aminométhyl)phényl)-4-isopropyl-2-hydroxybenzamide.

L'invention concerne également l'utilisation d'un composé tel que défini ci-avant pour la préparation d'une composition pharmaceutique destinée à la mise en œuvre d'une méthode de traitement du corps humain ou animal.

Par les termes « support acceptable sur le plan pharmaceutique », on entend tout support (par exemple excipient, substance, solvant, etc.) qui n'interfère pas avec l'efficacité de l'activité biologique du ou des actifs thérapeutiques présents et qui n'est pas toxique pour le sujet à qui la composition est administrée. Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, solvants, etc.

Les composés ou compositions selon l'invention peuvent être administrés de différentes manières et sous différentes formes. Ainsi, ils peuvent être injectés par voie orale ou systémique, comme par exemple par voie intraveineuse, intramusculaire, sous-cutanée, transdermique, intra-artérielle, etc. Pour les injections, les composés sont généralement conditionnés sous forme de suspensions liquides, qui peuvent être injectées au moyen de seringues ou de perfusions, par exemple. Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 1 μg et 2 g /administration, préférentiellement de 0,1 mg à 1 g /administration. Les administrations peuvent être quotidiennes ou répétées plusieurs fois par jour, le cas échéant.

D'autre part, la composition selon l'invention peut comprendre, en outre, au moins un autre agent ou principe actif thérapeutique. La composition pharmaceutique selon l'invention est plus spécifiquement pour une utilisation ou administration simultanée, séparée ou séquentielle du composé selon l'invention et d'au moins un autre agent ou principe actif thérapeutique, en particulier pour le traitement d'un désordre ou d'une maladie liée à un dérèglement de l'activité d'au moins une kallicréine, et en particulier la kallicréine 6.

Il s'agit avantageusement d'un composé ou d'une composition pharmaceutique selon l'invention pour le traitement d'un désordre ou d'une maladie liée à un dérèglement de l'activité d'au moins une kallicréine, et en particulier la kallicréine 6. Il a été trouvé de manière surprenante que les composés de formule (I) possèdent des propriétés inhibitrices réversibles de la kallicréine 6. Ils présentent également des propriétés inhibitrices non négligeables vis-à-vis de la plasmine et/ou de la kallicréine tissulaire (KLK1). Ils présentent en outre l'avantage de ne pas être neurotoxiques, en particulier vis-à-vis des neurones primaires et des oligodendrocytes.

Le composé de formule (I) ou la composition pharmaceutique selon l'invention sont particulièrement adaptés au traitement de maladies inflammatoires, neurodégénératives ou neuroinflammatoires.

Il s'agit en particulier du traitement de maladies choisies parmi l'ischémie cérébrale, la sclérose en plaques, la maladie de Parkinson, d'Alzheimer, des lésions de la moelle épinière, inflammation pulmonaire, complications vasculaires, en particulier liées au diabète.

L'autre agent ou principe actif thérapeutique est avantageusement un agent ou actif thérapeutique adapté au traitement tel que mentionné ci-dessus, et en particulier utilisable pour un traitement de maladies neurodégénératives ou neuroinflammatoires du système nerveux central, ou un traitement de maladies choisies parmi l'ischémie cérébrale, la sclérose en plaques, la maladie de Parkinson, d'Alzheimer, des lésions de la moelle épinière, inflammation pulmonaire, complications vasculaires, en particulier liées au diabète.

Par « traitement » ou « traiter », on entend selon l'invention une amélioration, une prophylaxie du désordre ou maladie, ou au moins un de ses symptômes. Ceci inclut l'amélioration ou la prévention d'au moins un paramètre physique mesurable du désordre ou maladie à traiter, qui n'est pas nécessairement discernable chez le sujet. Les termes « traitement » ou « traiter » se réfèrent également à l'inhibition ou le ralentissement de la progression de la maladie ou du désordre, ou la stabilisation d'un des symptômes de la maladie ou du désordre. Il peut s'agir aussi du retard du déclenchement d'au moins un symptôme de la maladie ou du désordre. Selon certains modes, les composés de l'invention sont administrés par mesure préventive. Dans ce contexte, les termes "traitement" ou "traiter" se réfèrent à la réduction du risque de développer la maladie ou le désordre.

## FIGURES

**Figure 1** : **Evaluation de la neurocytotoxicité des composés 1 (comparatif), 2 et 4** (selon l'invention). Le pourcentage de survie cellulaire a été déterminé en mesurant l'absorbance à 485 nm pour chaque concentration par rapport aux témoins traités uniquement avec du milieu complet (témoin positif) ou du DMSO 20 % (témoin négatif). Chaque concentration a été testée en quadruplicata.

**Figures 2 et 3: Evaluation de la cytotoxicité des composés 2 et 3 sur la lignée d'oligodendrocytes GFP/-mCherry.** La survie cellulaire a été évaluée par la mesure de l'intensité de fluorescence du marquage Hoechst rapporté au témoin basal (TB). Chaque concentration a été testée en quadruplicata. Statistiques : test de Mann-Whitney (p value = 0,019). **Figure 2** : Composé 2, **Figure 3** : Composé 3. Légende - TB : Témoin basal, T+ : témoin positif (acide 9-cis-rétinoïque).

**Figures 4-7: Evaluation de l'effet des composés 1 (comparatif), 2, 3 et 4 sur la différenciation des oligodendrocytes.** La différenciation des oligodendrocytes a été évaluée par la mesure de l'intensité de fluorescence du marquage mCherry, marqueur de la différenciation cellulaire, rapporté au témoin basal (TB). Chaque concentration a été testée en quadruplicata. Statistiques : test de Mann-Whitney (p value = 0,01). Légende - TB : Témoin basal, T+ : témoin positif (acide 9-cis-rétinoïque).

**Figure 8** : **Evaluation des composés 3 et 4 sur culture primaire d'OPCs de rat.** Les cellules ont été traitées par les composés **3** et **4** (3μM, 4μM, 5μM et 4μM, 6μM, 8μM respectivement). Après quantification du marquage MBP (cellules MBP+) par rapport au témoin basal (A), le composé 3 a un effet en faveur de la différenciation des OPCs à 5 μM (B). Le même résultat est observé pour le composé **4** à une concentration de 8 μM (C). Le pourcentage

(%) de cellules MBP positives **(MBP+)** après traitement par les composés **3** (5μM) et **4** (8μM) par rapport au témoin basal (D).

L'invention sera mieux comprise à l'aide des exemples qui suivent, lesquels se veulent illustratifs et non limitatifs.

## EXEMPLES

**Exemple** 1 : **Synthèse des Composés**

Schéma de synthèse général :

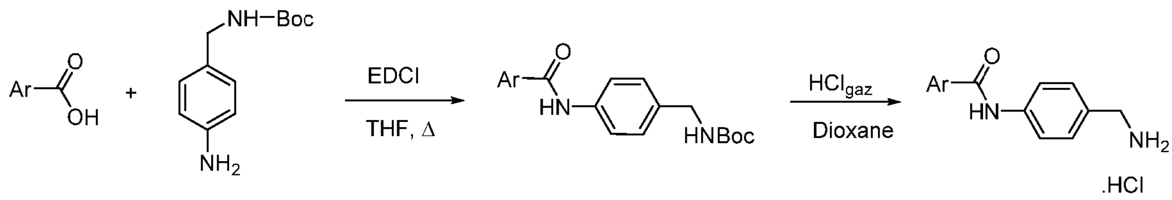


**7-12** **1-6**

Procédure générale pour la synthèse des composés **7-12** : A une solution de 100 mg du dérivé acide carboxylique approprié (0,45 mmol), dissous dans 2,5 mL de THF (tétrahydrofurane) sont ajoutés 103 mg d'EDCI (chlorhydrate de *N*-(3-diméthylaminopropyl)-*N'*-éthylcarbodiimide (0, 45 mmol, 1 équivalent) et 1 équivalent de *Tert*-butyl-(4-aminobenzyl)carbamate (0,45 mmol), synthétisé selon la méthode décrite par V. Famiglini et al., Eur. J. Med. Chem. 2014, 80, 101-111. La solution est agitée, puis portée à reflux pendant 12 heures. Après retour à température ambiante, la solution est évaporée à sec. Le résidu est dissous dans 20 mL d'acétate d'éthyle et la solution résultante est lavée par une solution aqueuse d'acide chlorhydrique 1N (3 × 20 ml), puis par 20 ml d'une solution aqueuse saturée d'hydrogénocarbonate de sodium. La phase organique est séchée sur sulfate de sodium anhydre, filtrée puis évaporée sous vide. Le résidu est ensuite purifié, soit par lavage (2 × 5 ml) par de l'éther diéthylique (composé **10),** soit par lavage (2 × 5 ml) par du dichlorométhane (composé **9)** soit chromatographié sur gel de silice pour les autres composés.

Tert-butyl (4-(4-méthyl-2-hydroxybenzoyl))aminobenzylcarbamate **(Composé 7)**

Eluant : nHex/AcOEt 1/2 v/v. Solide jaune pâle (masse : 131 mg, rendement : 82%); $^1$H NMR ($CDCl_3$, 400 MHz): δ ppm 1.45 (s, 9H), 2.33 (s, 3H), 4.28 (d, 2H, J = 5.5 Hz), 4.84 (bs, 1H), 6.70 (dd, 1H, J = 8.1 Hz, 1.1 Hz), 6.82 (d, 1H, J = 1.1 Hz), 7.27 (d, 2H, J = 8.4 Hz), 7.39 (d, 1H, J = 8.1 Hz), 7.50 (d, 2H, J = 8.4 Hz), 7.93 (bs, 1H), 11.94 (s, 1H); $^{13}$C NMR ($CDCl_3$, 100 MHz): δ ppm 21.9, 28.6, 44.4, 79.9, 112.1, 119.3, 120.3, 121.6, 125.5, 128.4, 136.1, 136.2, 146.1, 156.1, 162.1, 168.6; HPLC, Tr = 1.90 min; MS (ESI+): m/z 357.2 $[M+H]^+$, 379.1 $[M+Na]^+$, 301.2 $[M-tBu]^+$

Tert-butyl (4-(1-hydroxy-2-naphtoyl))aminobenzylcarbamate **(Composé 8)**

Elution : nHex/AcOEt 3/1 v/v; Solide blanc (masse : 40 mg, rendement: 23%); $^1$H NMR (DMSO d6, 300 MHz): δ ppm 1.40 (s, 9H), 4.13 (d, 2H, J = 5.9 Hz), 7.27 (d, 2H, J = 8.1 Hz), 7.40 (bt, 1H, J = 5.9 Hz), 7.46 (d, 1H, J = 8.9 Hz), 7.58 (dd, 1H, J = 8.1 Hz, 7.1 Hz), 7.68 (m, 3H), 7.92 (d, 1H, J = 8.1 Hz), 8.10 (d, 1H, J = 9.0 Hz), 8.31 (d, 1H, J = 8.1 Hz), 10.92 (bs, 1H); $^{13}$C NMR (DMSO $d_6$, 75 MHz): δ ppm 28.2, 43.0, 77.8, 107.5, 117.7, 121.1, 123.0, 124.7, 125.9, 127.2, 127.5, 129.1, 136.0, 136.1, 136.7, 155.8, 160.0, 169.4; ; HPLC, Tr= 2.13 min; MS (ESI+): m/z 415.1 $[M+Na]^+$, 337.0 $[M-tBu]^+$

Tert-butyl (4-(3-hydroxy-2-naphtoyl))aminobenzylcarbamate **(Composé 9)**

Solide blanc (masse : 57 mg, rendement: 32%); $^1$H NMR (DMSO $d_6$, 400 MHz): δ ppm 1.40 (s, 9H), 4.11 (d, 2H, J = 6.1 Hz), 7.25 (d, 2H, J = 8.4 Hz), 7.32 (s, 1H), 7.35 (m, 2H), 7.50 (ddd, 1H, J = 8.4 Hz, J = 6.8 Hz, J = 1.0 Hz), 7.69 (d, 2H, J = 8.4 Hz), 7.75 (d, 1H, J = 8.2 Hz), 7.92 (d, 1H, J = 8.2 Hz), 8.51 (s, 1H), 10.65 (bs, 1H); $^{13}$C NMR (DMSO $d_6$, 100 MHz): δ ppm 28.2, 43.0, 77.7, 110.6, 120.5, 121.6, 123.7, 125.7, 126.8, 127.4, 128.1, 128.7 130.4, 135.8, 135.9, 137.0, 154.0, 155.8, 165.6; HPLC, Tr= 1.99 min; MS (ESI+): m/z 415.2 $[M+Na]^+$, 337.1 $[M-tBu]^+$

Tert-butyl (4-(6-méthoxy-1-hydroxy-2-naphtoyl))aminobenzylcarbamate **(Composé 10)**

Solide blanc (masse : 34 mg, rendement 18%); $^1$H NMR ($CDCl_3$, 300 MHz): δ ppm 1.48 (s, 9H), 3.94 (s, 3H), 4.30 (d, 2H, J = 5.4 Hz), 4.87 (bs, 1H), 7.07 (d, 1H, J = 2.3 Hz), 7.16 (dd, 1H, J = 9.1 Hz, 2.4 Hz), 7.20 (d, 1H, J = 8.1

Hz), 7.30 (d, 2H, J = 8.3 Hz), 7.44 (d, 1H, J = 9.1 Hz), 7.55 (d, 2H, J = 8.3 Hz), 7.98 (bs, 1H), 8.35 (d, 1H, *J* = 9.1 Hz); $^{13}$C NMR ($CDCl_3$, 75 MHz): δ ppm 28.6, 44.4, 55.5, 79.3, 105.4, 106.2, 117.6, 118.2, 121.0, 121.6, 121.7, 125.9, 128.4, 136.0, 136.2, 138.6, 142.7, 143.1, 160.6, 161.6; HPLC, Tr= 2.14 min; MS (ESI+): m/z 445.2 $[M+Na]^+$, 367.1 $[M-tBu]^+$

*Tert*-butyl (4-(5-*tert*-butyl-2-hydroxybenzoyl))aminobenzylcarbamate **(Composé 11)**

Elution : DCM/EtOH 98.5/1.5 v/v; Solide blanc (masse : 58 mg, rendement: 32%); $^1$H NMR ($CDCl_3$, 300 MHz): δ ppm 1.31 (s, 9H), 1.44 (s, 9H), 4.26 (d, 2H, *J* = 5.9 Hz), 4.89 (bs, 1H), 6.93 (d, 1H, *J* = 8.7 Hz), 7.24 (m, 2H), 7.48 (m, 4H), 8.24 (bs, 1H), 11.0 (s, 1H); 13C NMR ($CDCl_3$, 75 MHz): δ ppm 28.6, 31.4, 31.6, 44.4, 79.9, 114.4, 118.4, 121.9, 122.1, 128.2, 132.3, 136.0, 136.1, 142.0, 156.1, 159.3, 168.7; HPLC, Tr= 2.10 min; MS (ESI+): m/z 399.3 $[M+H]^+$

Tert-butyl (4-(4-*isopropyl*-2-hydroxybenzoyl))aminobenzylcarbamate **(Composé 12)**

Elution : nHex/AcOEt 3/1 v/v; Solide blanc (masse : 54 mg, rendement: 31%); $^1$H NMR ($CDCl_3$, 300 MHz): δ ppm 1.60 (d, 6H, *J* = 6.7 Hz), 1.83 (s, 9H), 3.24 (hept, 1H, *J* = 6.7 Hz), 4.64 (d, 2H, *J* = 4.0 Hz), 5.25 (bs, 1H), 7.12 (d, 1H, *J* = 7.1 Hz), 7.60 (d, 2H, *J* = 7.6 Hz), 7.86 (m, 3H), 8.47 (bs, 1H), 12.30 (bs, 1H); $^{13}$C NMR ($CDCl_3$, 100 MHz): δ ppm 23.7, 28.6, 34.4, 44.4, 79.9, 112.5, 116.5, 117.9, 121.7, 125.8, 128.3, 136.0, 136.2, 156.2, 156.9, 162.1, 168.6; HPLC, Tr= 2.09 min; MS (ESI+): m/z 407.1 $[M+Na]^+$, 385.2 $[M+H]^+$

Procédure générale pour la synthèse des composés **1-6**

0.2 mmol de composé **7-12** sont dissous dans 5 mL d'une solution d'acide chlorhydrique 4N dans le 1,4-dioxane. La solution est agitée à température ambiante pendant 1 heure 30. Le dioxane est évaporé sous pression réduite et le résidu est lavé avec de l'éther diéthylique pour donner les composés **1-6.**

Chlorhydrate de *N*-(4-(aminométhyl)phényl)-5-*tert*-butyl-2-hydroxybenzamide **(Composé 1** - exemple comparatif) - composé 8 décrit dans Liang et al. (Bioorganic & Medicinal Chemistry letters, 22 (2012), 2450-2455)

Solide blanc. Masse : 52 mg, Rendement : 73%; $^1$H NMR (DMSO $d_6$, 300 MHz): δ ppm 1.20 (s, 9H), 3.93 (d, 2H, *J* = 5.2 Hz), 4.31 (bs, 2H), 6.81 (d, 1H, *J* = 8.4 Hz), 7.15 (m, 3H), 7.42 (m, 3H), 8.45 (bs, 3H), 10.43 (s, 1H); $^{13}$C NMR (DMSO $d_6$, 75 MHz): δ ppm 31.4, 33.7, 41.7, 46.3, 114.7, 119.0, 120.0, 123.2, 125.8, 127.5, 130.0, 130.3, 140.9, 153.3; HPLC, Tr= 1.39 min; MS (ESI+): m/z 285.2 $[M+H]^+$

Chlorhydrate de *N*-(4-(aminométhyl)phényl)-1-hydroxy-2-naphtamide **(Composé 2)**

Solide blanc. Masse : 63 mg, Rendement quantitatif, $^1$H NMR (DMSO $d_6$, 300 MHz): δ ppm 4.02 (s, 2H), 7.46 (d, 1H, *J* = 8.9 Hz), 7.53 (d, 2H, *J* = 8.3 Hz), 7.59 (d, 1H, *J* = 7.5 Hz), 7.68 (t, 1H, *J* = 7.5 Hz), 7.80 (d, 2H, *J* = 8.3 Hz), 7.92 (d, 1H, *J* = 8.0 Hz), 8.22 (d, 1H, *J* = 8.9 Hz), 8.31 (d, 1H, *J* = 8.3 Hz), 8.48 (bs, 3H), 10.43 (s, 1H); $^{13}$C NMR (DMSO $d_6$, 75 MHz): δ ppm 41.8, 107.5, 117.8, 122.1, 123.1, 123.2, 124.6, 125.9, 127.5, 129.2, 129.4, 130.2, 136.0, 137.8, 160.0, 169.6; HPLC, Tr= 1.45 min; MS (ESI+): m/z 294.1 $[M+H+1]^+$

Chlorhydrate de *N*-(4-(aminométhyl)phényl)-3-hydroxy-2-naphtamide **(Composé 3)**

Solide blanc. Masse : 38 mg, Rendement : 61%); $^1$H NMR (DMSO $d_6$, 400 MHz): δ ppm 4.00 (s, 2H), 7.37 (m, 2H), 7.51 (m, 3H), 7.76 (d, 1H, *J* = 8.3 Hz), 7.80 (d, 2H, *J* = 8.4 Hz), 7.94 (d, 1H, *J* = 8.3 Hz), 8.35 (bs, 3H), 8.53 (s, 1H), 10.70 (s, 1H), 11.39 (bs, 1H); 13C NMR (DMSO $d_6$, 100 MHz): δ ppm 41.8, 110.6, 120.4, 121.8, 123.7, 125.8, 126.9, 128.1, 128.7, 129.4, 129.6, 130.6, 135.8, 138.7, 153.6, 165.6; HPLC, Tr= 1.26 min; MS (ESI+): m/z 294.2 $[M+H+1]^+$

Chlorhydrate de *N*-(4-(aminométhyl)phényl)-4-méthyl-2-hydroxybenzamide **(Composé 4)**

Solide beige (masse : 53 mg, rendement : 90%); 1H NMR (DMSO $d_6$, 400 MHz): δ ppm 2.30 (s, 3H), 3.98 (q, 2H, *J* = 5.6 Hz), 6.78 (d, 1H, *J* = 8.2 Hz), 6.84 (s, 1H), 7.48 (d, 2H, *J* = 8.4 Hz), 7.74 (d, 2H, *J* = 8.4 Hz), 7.94 (d, 1H, *J* = 8.2 Hz), 8.43 (bs, 3H), 10.46 (s, 1H); $^{13}$C NMR (DMSO $d_6$, 100 MHz): δ ppm 21.1, 41.8, 114.2, 117.5, 120.1, 120.9, 129.0, 129.5, 130.3, 138.3, 144.4, 158.8, 166.7; HPLC, Tr= 1.11 min; MS (ESI+): m/z 258.0 $[M+H+1]^+$

Chlorhydrate de *N*-(4-(aminométhyl)phényl)-6-méthoxy-1-hydroxy-2-naphtamide **(Composé 5)**

Solide beige (masse : 65 mg, rendement : 91%); $^1$H NMR (DMSO $d_6$, 300 MHz): δ ppm 3.91 (s, 3H), 4.02 (s, 2H), 7.19 (dd, 1H, *J* = 9.2, 2.5 Hz), 7.35 (m, 2H), 7.51 (d, 2H, *J* = 8.6 Hz), 7.77 (d, 2H, *J* = 8.6 Hz), 8.14 (d, 1H, *J* = 9.2 Hz), 8.20 (d, 1H, *J* = 9.2 Hz), 8.36 (bs, 3H), 10.55 (bs, 1H), 14.00 (s, 1H); $^{13}$C NMR (DMSO $d_6$, 75 MHz): δ ppm 41.8,

55.4, 105.7, 106.4, 117.0, 117.9, 119.4, 122.0, 123.9, 125.0, 129.4, 130.1, 137.9, 138.1, 159.9, 160.3, 169.7; HPLC, Tr= 1.36 min; MS (ESI+): m/z 324.2 $[M+H]^+$

Chlorhydrate de *N*-(4-(aminométhyl)phényl)-4-isopropyl-2-hydroxybenzamide **(Composé 6)**

Solide blanc (masse : 41 mg, rendement : 56%); $^1$H NMR (DMSO $d_6$, 400 MHz): $\delta$ ppm 1.20 (d, 6H, $J$ = 6.8 Hz), 2.87 (hept, 1H, $J$ = 6.8 Hz), 3.99 (d, 2H, $J$ = 3.9 Hz), 6.87 (m, 2H), 7.48 (d, 2H, $J$ = 8.3 Hz), 7.74 (d, 2H, $J$ = 8.3 Hz), 7.95 (d, 1H, $J$ = 7.9 Hz), 8.39 (bs, 3H), 10.5 (s, 1H), 11.97 (bs, 1H); $^{13}$C NMR (DMSO $d_6$, 100 MHz): $\delta$ ppm 23.3, 33.3, 41.8, 114.6, 114.7, 117.4, 120.8, 129.0, 129.4, 138.3, 155.0, 158.8, 166.7; HPLC, Tr= 1.33 min; MS ($ESI^+$): m/z 286.1 $[M+H+1]^+$

**Exemple 2** : **Etudes des composés** - **Activités sur la KLK6, KLK1 et la plasmine**

Le point commun entre la kallicréine 6 (KLK6), la plasmine et la kallicréine tissulaire (KLK1) est leur capacité à activer les récepteurs PAR (Protease Activated Receptor) en clivant la partie *N*-terminale de ces derniers. Les récepteurs PAR, en particulier les récepteurs PAR1 et PAR2, sont connus pour être exprimées dans les cellules immunitaires. Ainsi, l'activation incontrôlée de ces derniers contribue aux processus inflammatoires.

**Matériels et méthodes**

**1. Procédure expérimentale**

Un test miniaturisé a été mis au point pour chaque protéase étudiée. Les conditions utilisées pour les criblages de molécules dans des plaques de 96 puits permettent une bonne reproductibilité des mesures d'activité sur de très petits volumes en utilisant le tampon Tris-HCl 50 mM, Citrate de sodium 1 M Brij-35 0,05 % (v/v) et les substrats peptidiques fluorogènes Boc-VPR-AMC, Boc-QAR-AMC, Boc-PFR-AMC à 37 °C et à pH 7,0.

Dans un test typique, 100$\mu$L de milieu réactionnel contient le tampon, 1 $\mu$L d'enzyme recombinante, 1 $\mu$L de substrat et un 1 $\mu$L du composé testé à 10 $\mu$M (dans le contrôle, 1 $\mu$L de DMSO). Dans chaque puits, l'inhibiteur est déposé en présence du mélange enzyme-tampon pour une pré-incubation de 15 minutes à 37°C. La réaction enzymatique est ensuite déclenchée par l'ajout du mélange tampon-substrat (100 $\mu$M). La libération du groupe fluorescent 7-amino-4-méthylcoumarine (AMC, $\lambda_{ex}$= 360 nm, $\lambda_{ém}$ = 460 nm) suite à l'hydrolyse du substrat catalysée par l'enzyme est suivie pendant 30 minutes à 37°C à l'aide d'un spectrofluorimètre FLUOstar.

D'autres substrats fluorogènes FRET mimant la partie $N_{ter}$ des récepteurs couplés aux protéines G activés par les protéases (Protease-Activated Receptors ou PARs) ont été employés pour la KLK6 (PAR-2) et la plasmine (PAR-4) dans les mêmes conditions en utilisant les longueurs d'onde ($\lambda_{ex}$ = 320 nm, $\lambda_{ém}$=405 nm).

Tableau 1

| Protéase | Substrat (100 $\mu$M) | Tampon |
|---|---|---|
| KLK1 : 1,6 nM | Boc-PFR-↓AMC | Tris-Hcl 50mM, CaCl2 10 mM, NaCl 150 mM, Brij-35 0,05 %, pH 7,5 |
| KLK6 : 2 nM /10 nM | Boc-QAR-↓AMC / PAR-2 (Abz-SSKGR↓SLIGQ-EDDnp) | Tris-HCl 50mM, Citrate 1M, Brij-35 0,05 %, pH 7 |
| Plasmine : 2,6 nM / | Boc-QAR-↓AMC / PAR-4 (Abz-LPAPR↓GYPGQ-EDDnp) | Tris-HCl 50mM, Citrate 1M, Brij-35 0,05 %, pH 7 |

Les substrats fluorogènes Boc-QAR-AMC et Boc-PFR-AMC ont été achetés auprès de l'entreprise Bachem®. Les substrats FRET MBP1 (Abz-RPSQR↓HATQ-EDDnp), MBP2 (Abz-HPAR↓ TAHQ-EDDnp), MBP3 (Abz-YGGR↓ASDQ-EDDnp), PAR2 (Abz- SSKGR↓SLIGQ-EDDnp) et PAR4 (Abz-LPAPR↓GYPGQ-EDDnp) ont été achetés auprès de GL Biochem®. Les séquences de ces substrats peptidiques reproduisent les sites de clivages des substrats biologiques de la KLK6 (MBP, PAR2) et de la plasmine (PAR4) et ont été synthétisés à façon. Tous les substrats ont été préparés à la concentration de 10 mM ou 20 mM dans 100% de DMSO (Sigma-Aldrich®) et stockés à température ambiante à l'abri de la lumière.

## 2. Analyse et quantification des effets inhibiteurs

Pour évaluer l'effet inhibiteur des composés, le même protocole expérimental est utilisé mais en présence de concentrations variables des molécules à tester avant d'ajouter le substrat synthétique. Les molécules testées, en général insolubles dans l'eau, sont préalablement dissoutes dans le DMSO. Le pourcentage final de DMSO dans le tampon de mesure est au maximum de 3% (v/v) aussi bien dans le témoin (pas d'inhibiteur) qu'en présence d'inhibiteur, quelle que soit la concentration de celui-ci. Le pourcentage d'inhibition varie en fonction de la concentration d'inhibiteur selon l'équation 1. L'ajustement des points expérimentaux à cette équation permet la détermination de la valeur de l'$IC_{50}$ (Concentration en inhibiteur permettant d'inhiber 50% de l'activité enzymatique maximale).

$$\% \text{ Inhibition} = 100\ (v_0 - v_i)/v_0 = 100 \times [I]\ /\ (IC_{50} + [I])\ \text{(éq. 1)}$$

avec $v_0$, vitesse initiale en absence d'inhibiteur et vi, vitesse initiale en absence d'inhibiteur. Dans quelques cas, une variation sigmoïdale est observée correspondant à l'équation 2 :

$$\% \text{ Inhibition} = 100\ (v0-vi)/v0 = 100 \times [I]^{nH}\ /\ (IC_{50}^{nH} + [I]^{nH})\ \text{(éq. 2)}$$

avec $n_H$, nombre de Hill.

Les mesures de fluorescence des activités enzymatiques ont été effectuées à l'aide d'un spectrofluorimètre lecteur de plaques multipuits BMG FLUOstar piloté par le logiciel Optima®. Les mesures sont réalisées dans des plaques noires 96 puits COSTAR à fond plat. Les traitements mathématiques et statistiques des données ont été réalisés à l'aide du logiciel Kaleidagraph®. Les mesures de pH des tampons ont été réalisées à l'aide d'un pH-mètre METTLER TOLEDO SevenCompactTM muni d'un senseur thermique et d'un support de calibration.

La réversibilité de l'effet inhibiteur a été analysée par la méthode de dilution. Cette méthode permet de différencier un inhibiteur covalent irréversible d'un inhibiteur réversible. L'enzyme et l'inhibiteur (ou le témoin négatif DMSO) sont incubés 15 minutes à 37°C pour permettre la formation du complexe enzyme-inhibiteur (E-I). Ce dernier est alors dilué au 100ème dans un mélange tampon-substrat puis la mesure de l'activité est lancée sur 30 minutes à 37°C. La vitesse initiale obtenue pour le contrôle DMSO représente 100% de l'activité et sert de référence pour la quantification de l'activité résiduelle de l'enzyme en présence de l'inhibiteur. La concentration de l'inhibiteur est choisie, à partir des courbes $IC_{50}$, de manière à ce que l'enzyme soit inhibée à plus de 80% avant dilution pour être sûr que l'effet après dilution ne soit pas dû à un effet de l'inhibiteur sur l'enzyme. Si, après dilution, l'activité est restaurée à plus de 50%, l'inhibiteur est réversible. Afin de déterminer le type d'inhibition exercée sur l'enzyme, l'inhibiteur est mis en compétition avec le substrat vis-à-vis du site actif de l'enzyme. L'inhibiteur est pré-incubé à différentes concentrations (1/4 $IC_{50}$ ; 1/2 $IC_{50}$ ; $IC_{50}$ ; 2 $IC_{50}$ ; 4 $IC_{50}$) avec un mélange enzyme-tampon pendant 15 minutes pour permettre la formation du complexe E-I. La réaction est lancée sur 30 minutes à 37°C dès l'ajout du mélange tampon - substrat. Pour l'expérience de compétitivité, plusieurs concentrations de substrat sont testées.

A la suite de cette expérience, le type d'inhibition est déterminé par la méthode de Dixon. Cette dernière consiste en le tracé de la droite $1/Vi = f([I_0])$ pour chaque concentration en substrat. Ces graphiques permettent de déterminer le type d'inhibition (compétitive, non compétitive ou incompétitive) ainsi que la constante d'inhibition Ki à partir du point d'intersection des différentes droites.

Les composés ont également été évalués sur la plasmine et sur la KLK1. Les tampons et substrats AMC utilisés sont présentés dans le Tableau 1.

## 3. Résultats

Les molécules ont donc été testées dans un premier temps à 10 μM pour évaluer leur effet inhibiteur sur l'activité enzymatique de la KLK6. Les % d'inhibition ont été déterminés à partir de l'activité résiduelle de l'hydrolyse du substrat fluorogène Boc-QAR-AMC (100 μM) par la KLK6 (2 nM) après 15 minutes d'incubation avec chaque molécule à 10 μM. Les composés donnant lieu à des pourcentages d'inhibition inférieur à 50% sont considérés non inhibiteurs. Les résultats sont présentés au Tableau 2 ci-dessous.

Tableau 2

| Composé | % Inhibition à 10 μM |
|---|---|
| 1 | 51,02 |

(suite)

| Composé | % Inhibition à 10 μM |
|---|---|
| 2 | 77,17 |
| 3 | 88,64 |
| 4 | 68,83 |
| 5 | 65 |
| 6 | 84,68 |

Le composé 1 ci-dessus (décrit dans Liang et al., Bioorganic & Medicinal Chemistry letters, 22 (2012), 2450-2455) à 10 μM entraîne donc un % d'inhibition de l'activité de la KLK6 inférieur à celui observé pour les composés selon l'invention.

Les pouvoirs inhibiteurs des composés ainsi que leur mécanisme d'action vis-à-vis de la KLK6 ont été déterminés. Les résultats sont rassemblés dans le tableau 3 ci-dessous.

Tableau 3, ND : non déterminé

| **Substrat** | **Boc-QAR-AMC** | | | **MBP3** | **PAR2** |
|---|---|---|---|---|---|
| **Composé** | **Type d'inhibition** | **$IC_{50}$ (μM)** | **$K_i$ (μM)** | **$IC_{50}$(μM)** | **$IC_{50}$(μM)** |
| 1 | Réversible compétitif | (9,06 ± 0,65) | 4,44 | (39,82 ± 5,00) | (21,71 ± 1,09) |
| 2 | Réversible compétitif | (1,50 ± 0,13) | 2,26 | (7,89 ± 0,63) | (2,63± 0,14) |
| 3 | Réversible non compétitif | (1,17 ± 0,05) | 0,79 | (2,38 ± 0,09) | (1,23± 0,07) |
| 4 | Réversible non compétitif | (5,42 ± 0,35) | 1,92 | (8,34 ± 0,90) | (5,06± 0,91) |
| 5 | Réversible compétitif | (3,74 ± 0,29) | 1 | ND | ND |
| 6 | Réversible non compétitif | (1,56 ± 0,04) | 1,75 | ND | ND |

Les molécules ont été testées pour évaluer leur éventuel effet inhibiteur sur l'activité enzymatique de la KLK1. Les % d'inhibition ont été déterminés à partir de l'activité résiduelle de l'hydrolyse du substrat fluorogène Boc-PFR-AMC (100 μM) par la KLK1 (1,6 nM) après 15 minutes d'incubation à 37°C avec chaque molécule à 10 μM. Les résultats sont présentés au Tableau 4 ci-dessous, tous les inhibiteurs sont réversibles.

Tableau 4, « ni » non inhibiteur

| ***Composé*** | ***% Inhibition à 10μM*** | ***$IC_{50}$ (μM)*** |
|---|---|---|
| 1 | 32,83 | ni |
| 2 | 47,71 | ni |
| **3** | **77,04** | **(5,90 ± 0,37)** |
| **4** | **61,66** | **(39,27 ± 12,07)** |
| **5** | **46,53** | **(30,68 ± 4,75)** |
| **6** | **57,66** | **(8,44 ± 0,28)** |

La sélectivité des composés vis-à-vis de la plasmine a été évaluée. Les % d'inhibition ont été déterminés à partir de l'activité résiduelle de l'hydrolyse du substrat fluorogène Boc-QAR-AMC (100 μM) par la plasmine (2,6 nM) après 15 minutes d'incubation à 37°C avec chaque molécule à 10 μM. Les résultats sont présentés au Tableau 5 ci-dessous.

Tableau 5

| Composé | % Inhibition à 10 μM |
|---|---|
| 1 | 39,26 |

(suite)

| Composé | % Inhibition à 10 µM |
|---|---|
| 2 | 97,37 |
| 3 | 80,98 |
| 4 | 54,30 |
| 5 | 100 |
| 6 | 70,15 |

Les pouvoirs inhibiteurs et mécanismes d'inhibition de ces composés vis-à-vis de la plasmine ont par la suite été déterminés. Les résultats sont rassemblés dans le tableau 6 ci-dessous.

Tableau 6, ND : non déterminé, « ni » non inhibiteur

| **Substrat** | **Boc-QAR-AMC** | | | **PAR4** |
|---|---|---|---|---|
| **Composé** | **Type d'inhibition** | **$IC_{50}$ (µM)** | **$K_i$ (µM)** | **$IC_{50}$ (µM)** |
| 1 | Réversible Compétitif | (17,05 ± 1,56) | 14,4 | ni |
| 2 | Réversible Compétitif | (1,50 ± 0,13) | 1,51 | (4,92 ± 0,54) |
| 3 | Réversible Compétitif | (3,75 ± 0,11) | 1,32 | (3,25 ± 0,48) |
| 4 | Réversible Compétitif | (12,49 ± 0,34) | 4,66 | ni |
| 5 | Réversible Non compétitif | (2,72 ± 0,34) | 2,16 | ND |
| 6 | Réversible Compétitif | (4,78 ± 0,23) | 0,67 | ND |

**Conclusion**

Les composés selon l'invention inhibent donc efficacement et de manière réversible la KLK6, certains composés inhibent également la plasmine et/ou la KLK1.

**Exemple 3** : **Evaluations biologiques des composés** - **Etudes sur cultures primaires de neurones corticaux murins et sur lignée d'oligodendrocytes de rat mCherry**

**Matériels et méthodes**

*1 - Analyse de la cytotoxicité sur culture primaire de neurones corticaux murins*

Les neurones ont été prélevés sur les embryons au stade E16 de femelles Swiss. Les cellules sont ensemencées à une densité de 50 000 cellules/puits dans des plaques 96 puits transparentes stériles préalablement revêtues. Les cellules sont cultivées 48h dans le milieu DMEM complété avec 10% de sérum de veau fœtal (SVF), de la pénicilline, de la streptomycine, du N2 et du B27, à 37°C sous atmosphère humide avec 5% de $CO_2$. Les cellules sont ensuite mises en présence des composés décrits ci-dessus (10, 25 ou 50 µM) ou du témoin DMSO puis incubées pendant 48h. Le milieu est ensuite remplacé par 100 µL de XTT (0,3 $mg.mL^{-1}$) afin de procéder au test de viabilité cellulaire. Le XTT est un dérivé de sel de tétrazolium dont la réduction par les déshydrogénases mitochondriales des cellules viables fait apparaître une coloration jaune-orangée. L'activité des mitochondries est déterminée par mesure de l'absorbance à 485 nm. Le pourcentage de survie cellulaire est calculé par le rapport de l'absorbance en présence de l'inhibiteur par rapport à la condition contrôle (DMSO). La molécule est considérée comme cytotoxique lorsque le pourcentage de survie cellulaire est inférieur à 80%.

*2 -Evaluation de l'effet des composés sur la différenciation des oligodendrocytes*

La lignée utilisée est une lignée de cellule CG4, précurseurs d'oligodendrocytes (OPC) de rat, et doublement transduite par les gènes de la GFP sous le contrôle du promoteur CMV et du marqueur mCherry sous contrôle d'un promoteur spécifique des oligodendrocytes différenciés et matures. Le marqueur mCherry n'est exprimé qu'au stade

d'oligodendrocytes différenciés et permet ainsi de tester des composés capables d'induire une différenciation des OPCs. Les cellules ont également été marquées au Hoechst afin d'évaluer leur viabilité. Les cellules sont cultivées dans le milieu de prolifération décrit par Louis et al. (Louis et al., J Neurosci Res. 1992 Jan; 31(1):193-204.) lors de leur encensement, puis passées dans un milieu de différenciation (DMEM/F12, B27, N1, biotine, laminine). Les cellules sont traitées par différentes concentrations d'inhibiteur pendant 72h dans des plaques 96 puits, puis criblées au moyen d'un microscope inversé automatisé. Les contrôles expérimentaux utilisés sont un témoin basal contenant uniquement le milieu de différenciation et un témoin positif, l'acide 9-cis-rétinoïque, connu pour son effet à induire une différenciation des OPCs (Huang et al., Nature Neuroscience 14, 45-53 (2011)).

Dans un premier temps, la cytotoxicité des composés est évaluée grâce à la comparaison de l'intensité du marquage Hoechst pour chaque condition expérimentale vis-à-vis du témoin basal. Un effet cytotoxique est supposé lorsque cette intensité diminue de manière significative.

Par la suite, l'effet des composés sur la différenciation des OPCs est évalué. Pour cela, l'intensité du marqueur mCherry est quantifiée pour chaque condition expérimentale vis-à-vis du témoin basal. Un effet en faveur de la différenciation se traduit par une augmentation significative de la fluorescence mCherry, à l'inverse d'un effet en défaveur de la différenciation. Les résultats sont analysés à l'aide du test statistique de Mann-Whitney.

**Résultats**

Les résultats sont présentés dans les Figures 1-7.

- La neurocytotoxicité des composés **1, 2** et **4** à 10, 25 et 50 μM a été évaluée sur une culture primaire de neurones corticaux murins **(Figure 1).** Selon le test de survie cellulaire au XTT, les composés **1** (comparatif), **2** et **4** (composés selon l'invention) se sont révélés non cytotoxiques à une concentration de 10 μM (% survie cellulaire > 80%). A une concentration de 25 μM), seuls les composés **2** et **4 n'induisent** pas de neurocytotoxicité (% survie cellulaire > 90%). A une concentration de 25 μM, seul le composé **2** n'est pas neurocytotoxique (% survie cellulaire > 90%). Quelle que soit la concentration utilisée, le pourcentage de survie cellulaire après traitement par les composés **2** et **4** est supérieur à celui observé avec le composé **1** de référence. La neurocytotoxicité induite par le composé **1** suit un effet dose-dépendant.
- La cytotoxicité a été évaluée sur la lignée d'oligodendrocytes doublement transduite avec la GFP et le marqueur mCherry par quantification du marquage Hoechst. Plus précisément, l'intensité du marquage relatif à chaque concentration en inhibiteur a été normalisée par rapport à celle du témoin basal. Les résultats sont présentés dans les **Figures 2 et 3,** pour les composés **2** et **3,** respectivement. Lorsque les cellules sont traitées avec le composé **2** (Figure 2), aucune différence de marquage n'est constatée. Ainsi, le composé **2** n'est pas cytotoxique. Le composé **3** (Figure 3) induit quant à lui une diminution du marquage Hoechst par rapport au témoin basal à 5 μM reflétant une potentielle cytotoxicité. Une évaluation de la cytotoxicité a été également réalisée pour les composés **1** et **4.** Le composé **1** de référence présente une toxicité à des concentrations supérieures à 10 μM. Le composé **4** ne présente pas de toxicité entre 2 et 12 μM.
- L'effet des composés **1, 2, 3** et **4** sur la différenciation des oligodendrocytes a été évalué sur la même lignée cellulaire **(Figures 4-7,** respectivement). Plus précisément, cet effet a été déterminé par la quantification de l'intensité marquage mCherry pour chaque condition expérimentale par rapport à celle du témoin basal. Le traitement par le composé **2** n'induit aucune modification du marquage mCherry et ce, quelle que soit la concentration. Cet inhibiteur ne semble donc avoir aucun effet sur la différenciation. En revanche, pour les composés **3** et **4,** on observe une augmentation significative du marquage mCherry par rapport au témoin basal à une concentration de 5 et 8 μM, respectivement (pvalue = 0,01, test de Mann-Whitney). Les composés **3** et 4 favorisent donc la différenciation des OPCs en oligodendrocytes matures.

La KLK6 joue un rôle sur la dynamique de la différenciation des OPCs (*Oligodendrocyte precursor cells).* Les OPCs suivent un processus de maturation à l'issue duquel ils atteignent le stade final d'oligodendrocytes matures myélinisants. L'excès de KLK6 a une action double sur ces cellules. D'une part, il induit une réduction drastique de l'arborescence des oligodendrocytes, de l'autre, il induit un ralentissement du processus de maturation des OPCs, diminuant significativement le nombre d'oligodendrocytes matures myélinisants. Ainsi, au vu de leurs profils pharmacologiques et biologiques, les composés **3** et **4,** selon l'invention, semblent particulièrement adaptés au traitement de désordres ou maladies telles que définies précédemment.

**Exemple 4 : Evaluations biologiques des composés 3 et 4 - Etudes sur cultures primaires de précurseurs d'oligodendrocyte (OPCs) de rat**

**Matériels et méthodes**

Les cultures primaires de cellules gliales sont obtenues à partir de cerveaux de rat à P0/P1. Après avoir sacrifié les animaux et retiré les méninges, les hémisphères cérébraux sont dissociés enzymatiquement à l'aide de trypsine 0,25% durant 5 minutes. Après inactivation de la trypsine à l'aide de DMEM + 10% sérum de veau fœtal (SVF)/+1% de Pénicilline-Streptomycine et dissociation mécanique jusqu'à homogénéisation, la suspension cellulaire ainsi obtenue est filtrée à l'aide d'un tamis 70μm. Les cellules sont ensuite placées dans des flasques de cultures substratés en poly-ornithine (100μg/mL), contenant un milieu DMEM + 10% sérum de veau fœtal (SVF)/1% de Pénicilline-Streptomycine. Les flasques sont placées dans un incubateur (37°C, 5% CO2). Le milieu est renouvelé au quatrième jour de culture puis tous les deux jours durant 10 à 14 jours.

Les cultures secondaires d'oligodendrocytes sont obtenues à partir de ces cultures après deux étapes d'agitation (250 rpm, 37°C). La première étape d'agitation d'une heure permet d'éliminer les cellules microgliales, tandis que la seconde agitation de 18 heures permet de détacher les progéniteurs d'oligodendrocytes (OPCs) du tapis astrocytaire. Par la suite, le surnageant contenant les cellules est soumis à des étapes d'adhérences préférentielles afin de retirer les cellules microgliales ainsi que les astrocytes qui pourraient compromettre la pureté de la culture.

Les OPCs purifiés sont ensemencés sur un substrat de poly-ornithine à 100μg/ml à la densité de 1000 cellules/$cm^2$. Apres adhésion les cellules sont laissées durant 12 heures dans un milieu DMEM/F12 + 2% B-27, 1% de Pénicilline-Streptomycine, bFGF (25ng/ml) et PDGF-BB (10ng/ml).

Ces mêmes cellules sont ensuite placées en différentiation pour quatre jours dans un milieu DMEM/F12 + 2% B-27 en présence ou non des diverses molécules à tester.

**Résultats**

Les composés **3** et **4** ont été évalués sur une culture primaire d'OPCs de rat. Dans ce modèle, le taux de différenciation a été mesuré par la quantification du marqueur MBP. La MBP, ou *Myelin Basic Protein,* est le principal constituant de la myéline. La MBP n'est exprimée que par les oligodendrocytes ayant atteint un stade de maturation terminal et donc, ayant acquis leur propriété myélinisante. Les résultats sont présentés dans la **Figure 8.**

Les cellules ont été traitées par les composés **3** et **4** (3μM, 4μM, 5μM et 4μM, 6μM, 8μM respectivement). Après quantification du marquage MBP (cellules MBP+) par rapport au témoin basal (A), le composé **3** a un effet en faveur de la différenciation des OPCs à 5 μM (B). Le même résultat est observé pour le composé **4** à une concentration de 8 μM (C). Le pourcentage (%) de cellules MBP positives **(MBP+)** après traitement par les composés **3** (5μM) et **4** (8μM) par rapport au témoin basal (D).

Les composés 3 et 4 favorisent de façon significative la différenciation des OPCs à des concentrations de 5 μM et 8 μM respectivement. Ces résultats soulignent le potentiel pro-myélinisant de ces composés.

**Revendications**

1. Composé pour une utilisation dans le traitement d'un désordre ou d'une maladie liée à un dérèglement de l'activité d'au moins une kallicréine, ledit composé étant de formule (I) suivante :

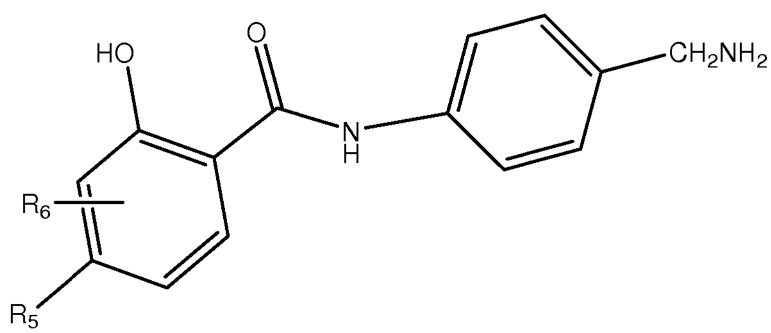

(I)

dans laquelle :

$R_5$ représente un radical (C1-C6)alkyl et $R_6$ représente un atome d'hydrogène ;
alternativement, $R_5$ et $R_6$ forment ensemble un cycle avec les deux carbones du cycle phényl auxquels ils sont rattachés pour former un groupe naphtyle, éventuellement substitué par au moins un atome d'halogène, un radical OH, (C1-C6)alkyle, (C1-C6)alkoxy, -C(O)$_2$R, ou - N(R)C(O)R', où R et R' étant indépendamment un atome d'hydrogène ou un radical ($C_1$-$C_6$)alkyl, de préférence au moins un radical (C1-C6)alkyle, (C1-C6)alkoxy ou un atome d'halogène,
la fonction amine pouvant présenter un groupement aminoprotecteur, ou un de ses sels pharmaceutiquement acceptable.

**2.** Composé pour une utilisation selon la revendication 1, où $R_5$ représente un radical alkyl, de préférence choisi parmi un radical méthyl, éthyl, n-propyl, isopropyl, n-butyl, et t-butyl, en particulier méthyl.

**3.** Composé pour une utilisation selon la revendication 1, dans lequel le composé est choisi parmi :

*N*-(4-(aminométhyl)phényl)-1-hydroxy-2-naphtamide,
*N*-(4-(aminométhyl)phényl)-3-hydroxy-2-naphtamide,
*N*-(4-(aminométhyl)phényl)-4-méthyl-2-hydroxybenzamide,
*N*-(4-(aminométhyl)phényl)-6-méthoxy-1-hydroxy-2-naphtamide, *N*-(4-(aminométhyl)phényl)-4-isopropyl-2-hydroxybenzamide,

et un de ses sels, en particulier le chlorhydrate, ou un de ses composés amino-protégés.

**4.** Composé pour une utilisation selon la revendication 1, dans lequel le composé est le N-(4-(aminométhyl)phényl)-1-hydroxy-2-naphtamide, *N*-(4-(aminométhyl)phényl)-4-méthyl-2-hydroxybenzamide, un de leurs sels, en particulier le chlorhydrate, ou un de leurs composés amino-protégés.

**5.** Composé *N*-(4-(aminométhyl)phényl)-4-isopropyl-2-hydroxybenzamide, un de ses sels, en particulier le chlorhydrate, ou un de ses composés amino-protégés.

**6.** Composition pharmaceutique comprenant, dans un milieu pharmaceutiquement acceptable, au moins un composé de formule (I) tel que défini dans l'une quelconque des revendications précédentes.

**7.** Composition selon la revendication 6, le composé de formule (I) étant choisi parmi :

*N*-(4-(aminomethyl)phenyl)-1-hydroxy-2-naphtamide,
*N*-(4-(aminomethyl)phenyl)-3-hydroxy-2-naphtamide,
*N*-(4-(aminométhyl)phényl)-4-méthyl-2-hydroxybenzamide,
*N*-(4-(aminométhyl)phényl)-6-méthoxy-1-hydroxy-2-naphtamide,
*N*-(4-(aminométhyl)phényl)-4-isopropyl-2-hydroxybenzamide,
un de ses sels, en particulier le chlorhydrate, ou un de ses composés amino-protégés.

**8.** Composition selon la revendication 6, le composé étant choisi parmi le N-(4-(aminométhyl)phényl)-1-hydroxy-2-naphtamide et le *N*-(4-(aminométhyl)phényl)-4-méthyl-2-hydroxybenzamide, plus spécifiquement le *N*-(4-(aminométhyl)phényl)-4-isopropyl-2-hydroxybenzamide.

**9.** Composé ou composition pour une utilisation selon l'une des revendications précédentes, dans lequel le désordre ou la une maladie est liée à un dérèglement de l'activité d'au moins une kallicréine qu'est la kallicréine 6.

**10.** Composé ou composition pour une utilisation selon l'une des revendications précédentes, pour une utilisation dans le traitement de maladies inflammatoires, neurodégénératives ou neuro-inflammatoires du système nerveux central.

**11.** Composé ou composition pour une utilisation selon l'une des revendications précédentes, pour une utilisation dans le traitement d'une maladie choisie parmi l'ischémie cérébrale, la sclérose en plaques, la maladie de Parkinson, d'Alzheimer, des lésions de la moëlle épinière, inflammation pulmonaire, complications vasculaires, en particulier liées au diabète

**12.** Composé ou composition pour une utilisation selon l'une des revendications précédentes, pour une utilisation dans le traitement de la sclérose en plaques.

## Patentansprüche

1. Verbindung zur Verwendung bei der Behandlung einer Störung oder einer Krankheit, die mit einer Fehlregulation der Aktivität von mindestens einem Kallikrein verbunden ist, wobei die Verbindung die folgende Formel (I) aufweist:

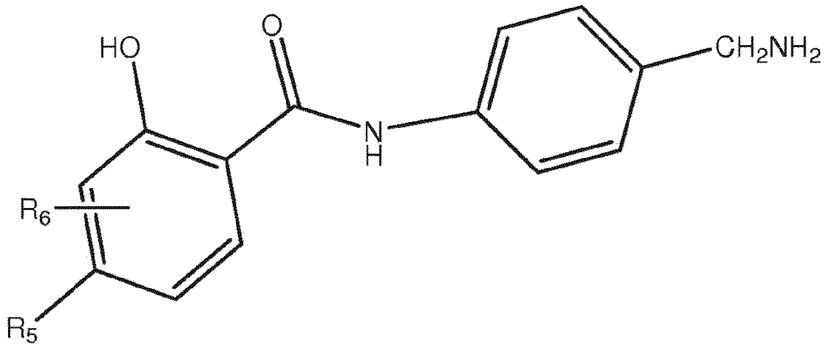

(I)

worin:

$R_5$ einen (C1-C6)-Alkylrest darstellt und $R_6$ ein Wasserstoffatom darstellt;
alternativ $R_5$ und $R_6$ zusammen einen Ring mit den beiden Kohlenstoffen des Phenylrings, an den sie gebunden sind, bilden, um eine Naphthylgruppe zu bilden,
die gegebenenfalls substituiert ist durch mindestens ein Halogenatom, ein OH, (C1-C6)-Alkyl, (C1-C6)-Alkoxy, $-C(O)_2R$ oder -N(R)C(O)R', wobei R und R' unabhängig voneinander ein Wasserstoffatom oder ein $(C_1-C_6)$-Alkylrest sind,
vorzugsweise mindestens ein (C1-C6)-Alkyl, (C1-C6)-Alkoxy oder ein Halogenatom,
wobei die Amin-Funktion möglicherweise eine Aminoschutzgruppe aufweist, oder
eines ihrer pharmazeutisch verträglichen Salze.

2. Verbindung zur Verwendung nach Anspruch 1, wobei $R_5$ für einen Alkylrest steht, vorzugsweise ausgewählt aus einem Methyl-, Ethyl-, n-Propyl-, Isopropyl-, n-Butyl- und t-Butylrest, insbesondere Methyl.

3. Verbindung zur Verwendung nach Anspruch 1, wobei die Verbindung ausgewählt ist aus:

*N*-(4-(Aminomethyl)phenyl)-1-hydroxy-2-naphthamid,
*N*-(4-(Aminomethyl)phenyl)-3-hydroxy-2-naphthamid,
*N*-(4-(Aminomethyl)phenyl)-4-methyl-2-hydroxybenzamid, *N*-(4-(Aminomethyl)phenyl)-6-methoxy-1-hydroxy-2-naphthamid,
*N*-(4-(Aminomethyl)phenyl)-4-isopropyl-2-hydroxybenzamid,

und eines seiner Salze, insbesondere das Hydrochlorid, oder eine seiner aminogeschützten Verbindungen.

4. Verbindung zur Verwendung nach Anspruch 1, wobei die Verbindung N-(4-(Aminomethyl)phenyl)-1-hydroxy-2-naphthamid, *N*-(4-(Aminomethyl)phenyl)-4-methyl-2-hydroxybenzamid ist, eines ihrer Salze, insbesondere das Hydrochlorid, oder eine ihrer aminogeschützten Verbindungen.

5. Verbindung N-(4-(Aminomethyl)phenyl)-4-isopropyl-2-hydroxybenzamid, eines seiner Salze, insbesondere das Hydrochlorid, oder eine seiner aminogeschützten Verbindungen

6. Pharmazeutische Zusammensetzung, umfassend in einem pharmazeutisch verträglichen Medium mindestens eine Verbindung der Formel (I), wie in einem der vorhergehenden Ansprüche definiert.

7. Zusammensetzung nach Anspruch 6, wobei die Verbindung der Formel (I)

*N*-(4-(Aminomethyl)phenyl)-1-hydroxy-2-naphthamid,
*N*-(4-(Aminomethyl)phenyl)-3-hydroxy-2-naphthamid,
*N*-(4-(Aminomethyl)phenyl)-4-methyl-2-hydroxybenzamid,

*N*-(4-(Aminomethyl)phenyl)-6-methoxy-1-hydroxy-2-naphthamid,
*N*-(4-(Aminomethyl)phenyl)-4-isopropyl-2-hydroxybenzamid,

eines seiner Salze, insbesondere das Hydrochlorid, oder eine seiner aminogeschützten Verbindungen.

**8.** Zusammensetzung nach Anspruch 6, wobei die Verbindung ausgewählt ist aus N-(4-(Aminomethyl)phenyl)-1-hydroxy-2-naphthamid und N-(4-(Aminomethyl)phenyl)-4-methyl-2-hydroxybenzamid, insbesondere N-(4-(Aminomethyl)phenyl)-4-isopropyl-2-hydroxybenzamid.

**9.** Verbindung oder Zusammensetzung zur Verwendung nach einem der vorhergehenden Ansprüche, wobei die Störung oder Krankheit mit einer Störung der Aktivität von mindestens einem Kallikrein, das das Kallikrein 6 ist, verbunden ist.

**10.** Verbindung oder Zusammensetzung zur Verwendung nach einem der vorhergehenden Ansprüche, zur Verwendung bei der Behandlung von entzündlichen, neurodegenerativen oder neuroinflammatorischen Erkrankungen des Zentralnervensystems.

**11.** Verbindung oder Zusammensetzung zur Verwendung nach einem der vorhergehenden Ansprüche zur Verwendung bei der Behandlung einer Krankheit ausgewählt aus zerebraler Ischämie, Multipler Sklerose, Parkinson-Krankheit, Alzheimer-Krankheit, Markläsionen des Rückenmarks, Lungenentzündung, vaskulären Komplikationen, insbesondere im Zusammenhang mit Diabetes.

**12.** Verbindung oder Zusammensetzung zur Verwendung nach einem der vorhergehenden Ansprüche zur Verwendung bei der Behandlung von Multipler Sklerose.

## Claims

**1.** Compound for use in the treatment of a disorder or disease related to a dysregulation of the activity of at least one kallikrein, said compound being of formula (1) below:

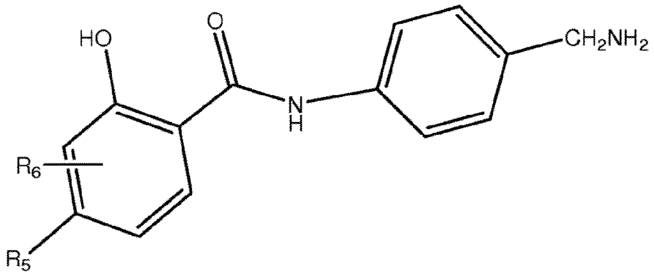

(I)

wherein:

$R_5$ represents a (C1-C6) alkyl radical and $R_6$ represents a hydrogen atom; alternatively, $R_5$ and $R_6$ together form a ring with the two carbons of the phenyl ring to which they are attached to form a naphthyl group, optionally substituted by at least one halogen atom, an OH, (C1-C6) alkyl, (C1-C6) alkoxy, $-C(O)_2R$, or - N(R)C(O)R' radical, where R and R' are, independently, a hydrogen atom or a (C1-C6) alkyl radical, preferably at least one (C1-C6) alkyl radical, (C1-C6) alkoxy radical or a halogen atom,
the amine function being able to have an amino protecting group, or a pharmaceutically acceptable salt thereof.

**2.** Compound for use according to claim 1, where $R_5$ represents an alkyl radical, preferably chosen from among a methyl, ethyl, n-propyl, isopropyl, n-butyl, and t-butyl radical, in particular a methyl radical.

**3.** Compound for use according to claim 1 wherein the compound is chosen from among:

*N*-(4-(aminomethyl)phenyl)-1-hydroxy-2-naphthamide,
*N*-(4-(aminomethyl)phenyl)-3-hydroxy-2-naphthamide,

*N*-(4-(aminomethyl)phenyl)-4-methyl-2-hydroxybenzamide,
*N*-(4-(aminomethyl)phenyl)-6-methoxy-1-hydroxy-2-naphthamide,
*N*-(4-(aminomethyl)phenyl)-4-isopropyl-2-hydroxybenzamide, and a salt thereof,

in particular hydrochloride, or an amino-protected compound thereof.

**4.** Compound for use according to claim 1 wherein the compound is *N*-(4-(aminomethyl)phenyl)-1-hydroxy-2-naphthamide, *N*-(4-(aminomethyl)phenyl)-4-methyl-2-hydroxybenzamide, a salt thereof, in particular hydrochloride, or an amino-protected compound thereof.

**5.** *N*-(4-(aminomethyl)phenyl)-4-isopropyl-2-hydroxybenzamide compound, a salt thereof, in particular hydrochloride, or an amino-protected compound thereof.

**6.** Pharmaceutical composition comprising, in a pharmaceutically-acceptable medium, at least one compound of formula (1) as defined in any one of the preceding claims.

**7.** Composition according to claim 6, the compound of formula (1) being chosen from among:

*N*-(4-(aminomethyl)phenyl)-1-hydroxy-2-naphthamide,
*N*-(4-(aminomethyl)phenyl)-3-hydroxy-2-naphthamide,
*N*-(4-(aminomethyl)phenyl)-4-methyl-2-hydroxybenzamide,
*N*-(4-(aminomethyl)phenyl)-6-methoxy-1-hydroxy-2-naphthamide,
*N*-(4-(aminomethyl)phenyl)-4-isopropyl-2-hydroxybenzamide,

a salt thereof, in particular hydrochloride, or an amino-protected compound thereof.

**8.** Composition according to claim 6, the compound being chosen from among *N*-(4-(aminomethyl)phenyl)-1-hydroxy-2-naphthamide and *N*-(4-(aminomethyl)phenyl)-4-methyl-2-hydroxybenzamide, more specifically *N*-(4-(aminomethyl)phenyl)-4-isopropyl-2- hydroxybenzamide.

**9.** Compound or composition for a use according to any one of the preceding claims, wherein the disorder or disease is linked to a dysregulation of the activity of at least one kallikrein, which is kallikrein 6.

**10.** Compound or composition for a use according to any one of the preceding claims, for use in the treatment of inflammatory, neurodegenerative or neuroinflammatory diseases of the central nervous system.

**11.** Compound or composition for a use according to any one of the preceding claims, for use in the treatment of a disease chosen from among cerebral ischemia, multiple sclerosis, Parkinson's disease, Alzheimer's disease, spinal cord damage, lung inflammation or vascular complications, especially related to diabetes

**12.** Compound or composition for a use according to any one of the preceding claims, for use in the treatment of multiple sclerosis.

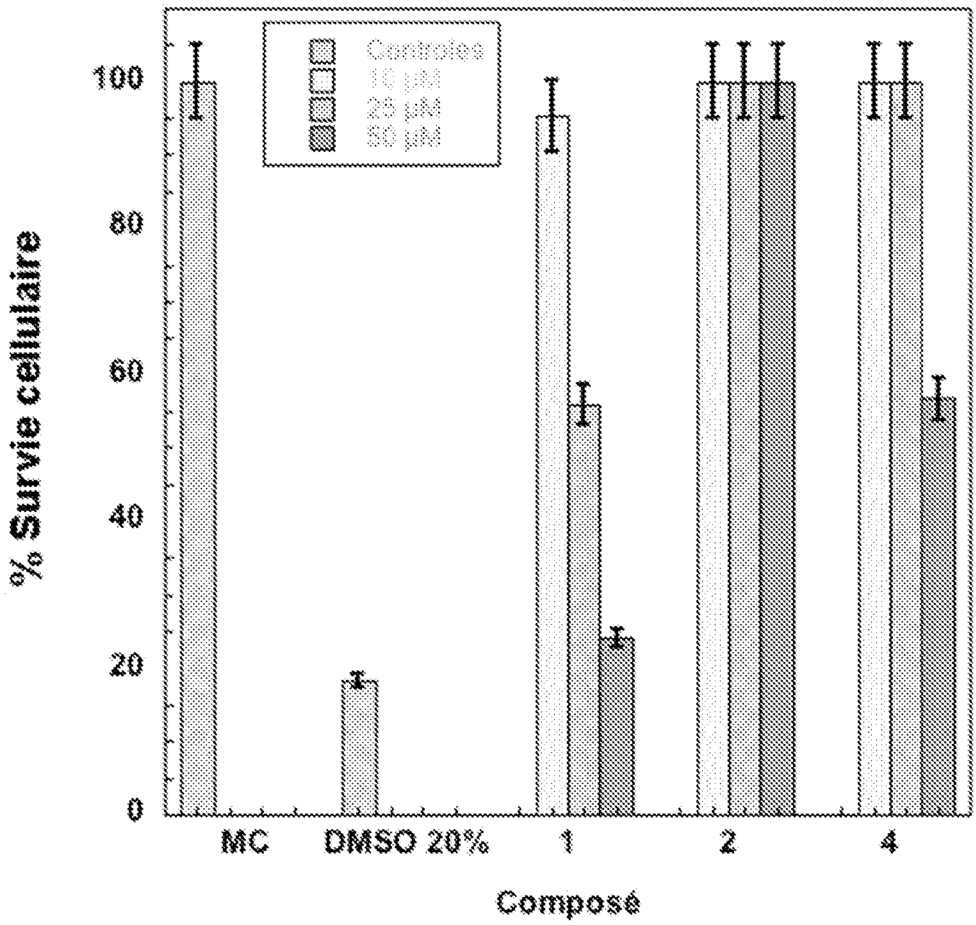
Controles
10 µM
25 µM
50 µM
% Survie cellulaire
0
20
40
60
80
100
MC
DMSO 20%
1
2
4
Composé

Figure 1

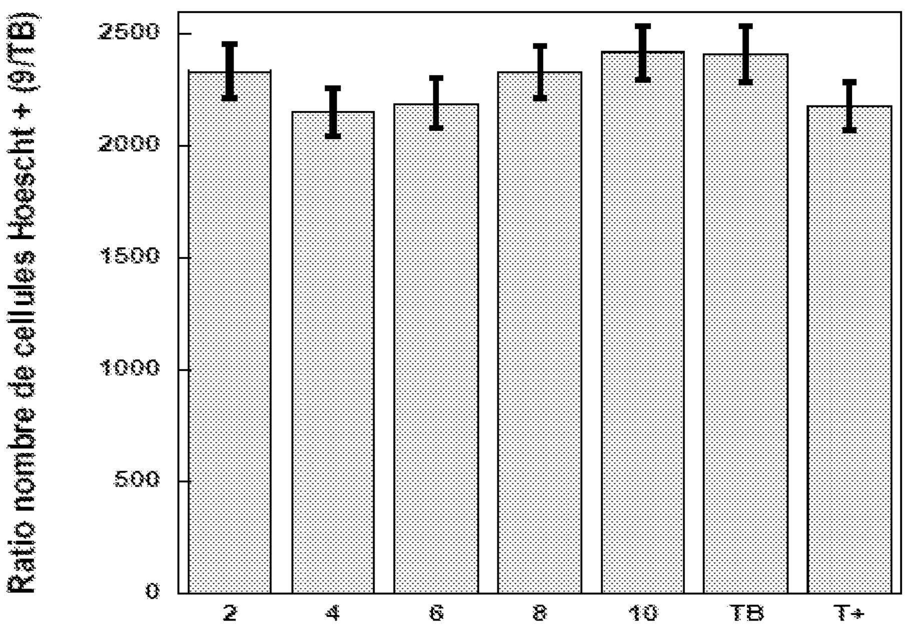


Composé 2 (µM)

**Figure 2**

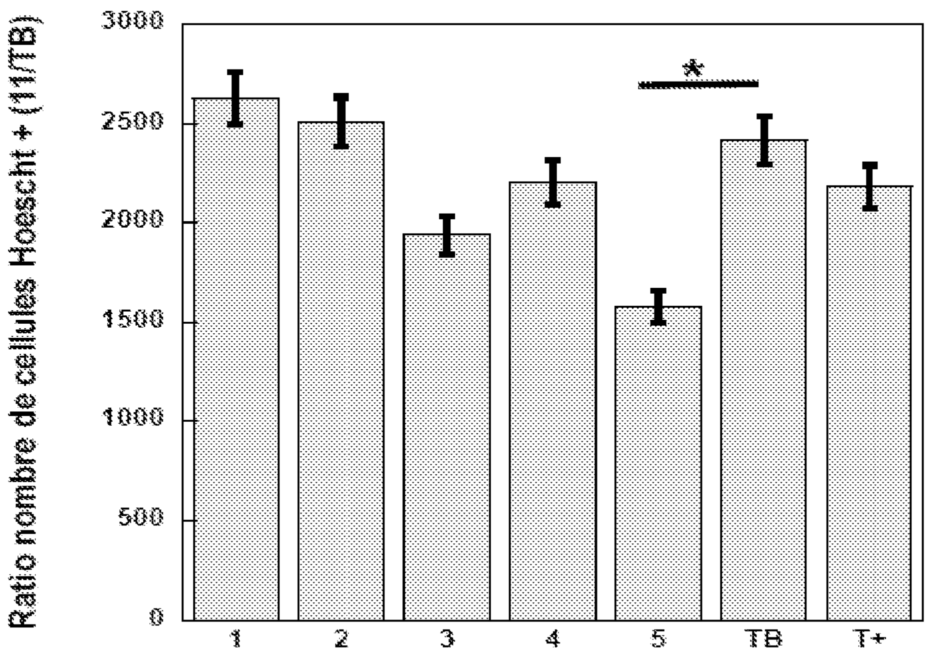


Composé 3 (µM)

**Figure 3**

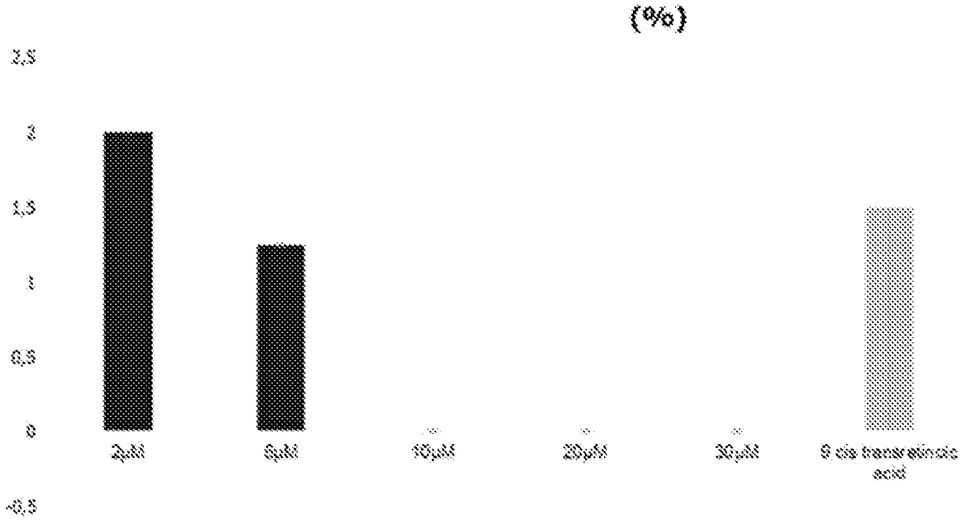


Composé 1

**Figure 4**

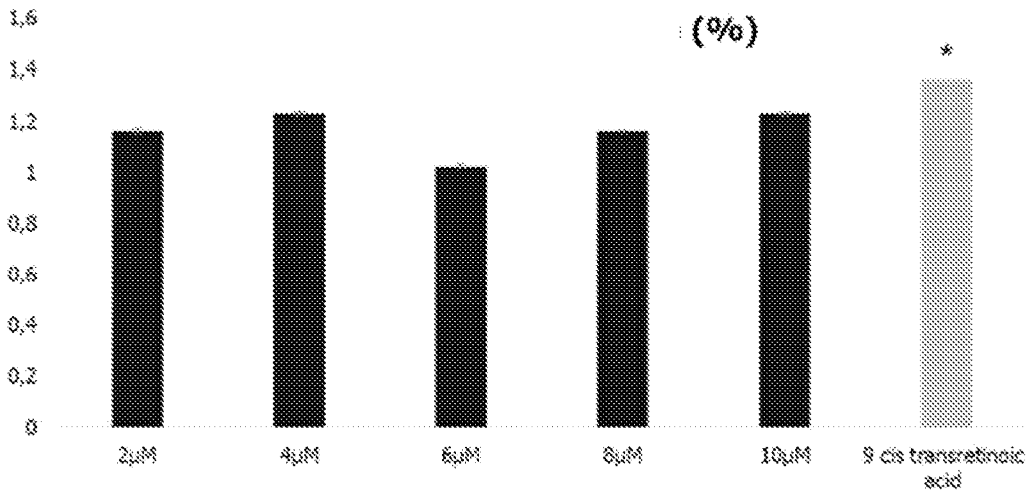


Composé 2

**Figure 5**

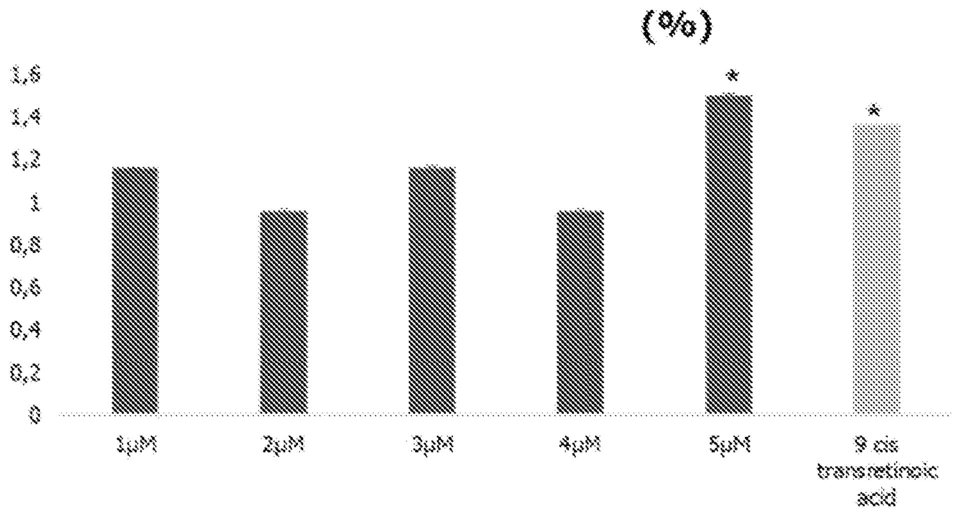


Composé 3

**Figure 6**

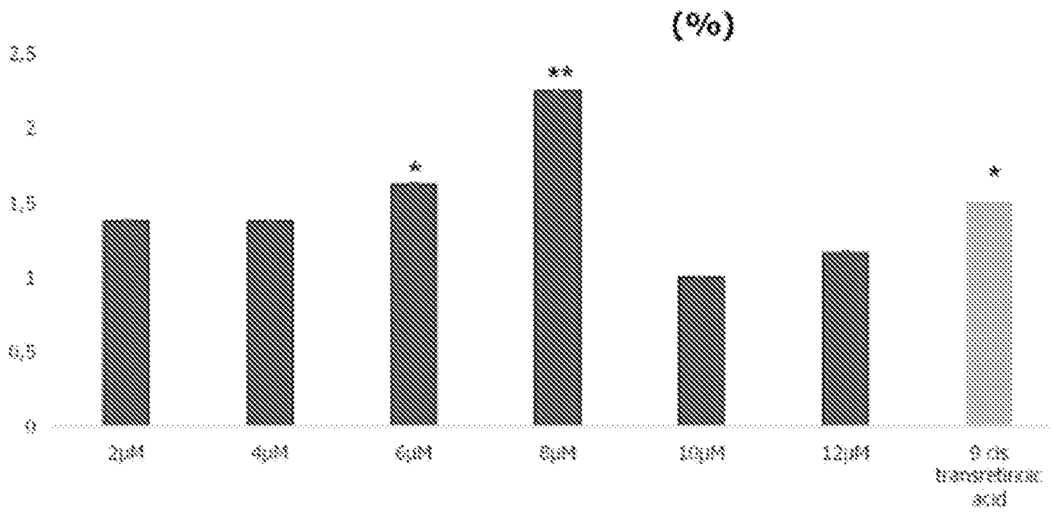


Composé 4

**Figure 7**

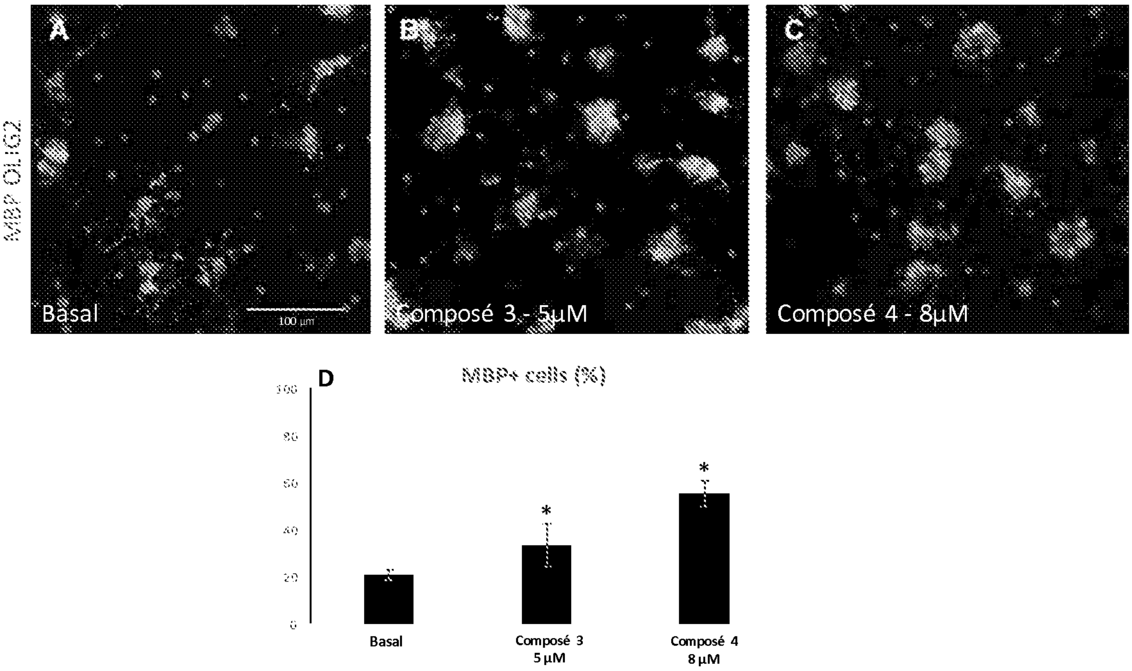
MBP OLIG2
A
B
C
Basal
100 µm
Composé 3 - 5µM
Composé 4 - 8µM
D
MBP+ cells (%)
100
80
60
40
20
0
*
*
Basal
Composé 3
5 µM
Composé 4
8 µM

**Figure 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **LIANG et al.** Human kallikrein 6 inhibitors with a para-amidobenzylamine P1 group identified through virtual screening. *Bioorganic & Medicinal Chemistry letters,* 2012, vol. 22, 2450-2455
- *J. Pharm. Sci.,* 1977, vol. 66, 2
- Handbook of Pharmaceutical Salts: Properties, Selection, and Use. 2002
- **V. FAMIGLINI et al.** *Eur. J. Med. Chem.,* 2014, vol. 80, 101-111
- **LIANG et al.** *Bioorganic & Medicinal Chemistry letters,* 2012, vol. 22, 2450-2455
- **LOUIS et al.** *J Neurosci Res,* Janvier 1992, vol. 31 (1), 193-204
- **HUANG et al.** *Nature Neuroscience,* 2011, vol. 14, 45-53